# EUROPEAN PATENT APPLICATION

(11) **EP 1 334 716 A1**
(43) Date of publication of application: **13.08.2003**
(21) Application number: 01974848.2
(22) Date of filing: 15.10.2001
(51) Int. Cl.: A61K 9/20, A61K 9/16, A61K 47/10, A61K 47/26, A61K 31/51, A61K 31/455, A61K 31/167, A61K 31/405, A61K 31/135, A61K 31/4704, A61K 31/216, A61K 31/5513, A61K 31/515, A61K 31/606, A61K 31/43, A61K 31/513, A61K 31/401, A61K 31/4422, A61K 31/166, A61K 31/404

(54) **MEDICINAL COMPOSITIONS QUICKLY DISINTEGRATING IN THE ORAL CAVITY AND PROCESS FOR PRODUCING THE SAME**

(30) Priority: 16.10.2000 JP 2000314609
(71) Applicant: DAIICHI PHARMACEUTICAL CO., LTD., Chuo-ku, Tokyo 103-8234 (JP)
(72) Inventor: NAKAGAMI, Hiroaki, DAIICHI PHARMACEUTICAL CO.,LTD, Tokyo 134-8630 (JP); KUNO, Yoshio, DAIICHI PHARMACEUTICAL CO., LTD., Tokyo 134-8630 (JP)
(74) Representative: Wächtershäuser, Günter, Prof. Dr.
(86) International application number: JP0109026
(87) International publication number: WO02032403

(57) **Abstract**

This invention relates to a medicinal composition, which rapidly disintegrates when taken in the oral cavity and shows sufficient hardness upon production, distribution and use in usual manner, can be obtained by adding, to a sugar alcohol and/or saccharide, a sugar alcohol and/or saccharide having a lower melting point than the first-mentioned sugar alcohol and/or saccharide and then subjecting the resulting powder to combined processing of compression and heating. This invention can provide medicinal compositions, which rapidly disintegrate when taken in the oral cavity without water and are excellent in handling ease owing to exhibition of sufficient hardness upon their production, transportation and use in usual manner, and can also provide a process for the production of the medicinal compositions, which is simpler and can avoid contact between an active ingredient and water as needed.

## Description

### Technical Field

This invention relates to medicinal compositions, which rapidly disintegrate when taken in the oral cavity but show sufficient hardness upon production, transportation and use in usual manner, and also to a production process thereof.

### Background Art

Keeping in step with a move toward an aging society and changes in the living environment, there is now an outstanding desire for the development of medicinal preparations which are easy to handle and take for those aged, infants and patients restricted in water intake. Conventional solid preparations of medicines include preparation forms such as tablets, granules, powders, and capsules. These solid medicines, however, involve problems in that upon taking them, a great amount of water is needed and moreover, they are not palatable. These problems are serious especially when those aged, infants or patients restricted in water intake take them. Further, psychotic patients or the like may take such a behavior that, when tablets or the like are administered, they once take the tablets or the like in their mouths but, when left alone subsequently, spit them out and do not take them. With a view to providing solid preparations of medicines which rapidly disintegrate or dissolve even when taken in the oral cavity without water, numerous studies have therefore been reported on compositions and/or production processes both in Japan and abroad.

In the case of "Zydis® Fast Dissolving Dosage Forms", intraoral dissolving preparations commercially available from R.P. Scherer Ltd., or the intraoral disintegrating preparations disclosed in WO 93/12769 entitled "Intrabuccally Disintegrating Preparation and Production Thereof", for example, each preparation is produced by filling a solution or suspension of a medicinal ingredient in pockets of a PTP (Press Through Package) sheet formed beforehand and then freeze-drying or vacuum-drying the solution or suspension. However, this production process requires freeze drying or vacuum drying, resulting in higher production cost. It is also accompanied by additional drawbacks in that the resulting tablets are weak in strength and are so brittle that they cannot withstand usual transportation.

According to the invention disclosed in JP 11-116464 A entitled "Rapid Dissoluting Solid Preparations and Production Process Thereof", WO 93/15724 entitled "Fast Soluble Tablets" or JP 6-218028 entitled "Process and Apparatus for Forming Molded Tablets, and Molded Tablets", powder containing a medicinal ingredient and moistened with water is tableted, followed by drying. Such processes can provide tablets having excellent intraoral disintegration property and rather high hardness, but involve difficulty in controlling the weight of tablets because moistened powder is tableted. In addition, they require a special apparatus since production can hardly be performed with a conventional tableting machine.

On the other hand, JP 8-29105 A entitled "Production Process of Fast Dissolving Tablets, and Fast Dissolving Tablets Produced by the Production Process" or JP 11-12161 A entitled "Process for Producing Intraoral Rapid-Disintegrating Preparations" discloses a process for producing a target preparation, which comprises tableting dry-state powder into compacts at low pressure, moistening the compacts and then drying the moistened compacts. This process also includes many production steps, thereby involving a problem in the efficiency of production such as long production time.

However, the above-described production processes are all accompanied by a drawback that they can hardly be applied to a water-unstable active ingredient because the active ingredient is kept contacting water for a long time.

An object of the present invention is to provide a medicinal composition, which rapidly disintegrates when taken in the oral cavity and which shows sufficient hardness upon production, transportation and use in usual manner and is excellent in handling ease. Another object of the present invention is to provide a process for producing a medicinal composition more easily while permitting avoidance of contact between an active ingredient and water as needed.

### Disclosure of the Invention

As a result of extensive research, the present inventors have found that a medicinal composition, which rapidly disintegrates when taken in the oral cavity and shows sufficient hardness upon production, distribution and use in usual manner, can be obtained by adding, to a sugar alcohol and/or saccharide, a sugar alcohol and/or saccharide having a lower melting point than the first-mentioned sugar alcohol and/or saccharide and then subjecting the resulting powder to combined processing of mixing, compression and heating.

Described specifically, the present invention relates to a medicinal composition produced by at least mixing, compression and heating, wherein the medicinal composition comprises two or more of sugar alcohols and/or saccharides, and an active ingredient; and a difference between a melting point of one having a highest content of the two or more sugar alcohols and/or saccharides and that of any remaining one of the two or more sugar alcohols and/or saccharides is 5°C or greater.

The present invention also relates to a medicinal composition produced by at least mixing, compression and heating, wherein the medicinal composition comprises two or more of sugar alcohols and/or saccharides, and a water-unstable active ingredient; and a difference between a melting point of one having a highest content of the two or more sugar alcohols and/or saccharides and that of any remaining one of the two or more sugar alcohols and/or saccharides is 5°C or greater.

The present invention also relates to a medicinal composition produced by at least mixing, compression and heating, wherein the medicinal composition comprises two or more of sugar alcohols and/or saccharides, and an active ingredient whose melting point or decomposition point is 140°C or higher; and a difference between a melting point of one having a highest content of the two or more sugar alcohols and/or saccharides and that of any remaining one of the two or more sugar alcohols and/or saccharides is 5°C or greater.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; and the medicinal composition has a wetting time of 10 seconds or shorter when tested by a wetting test.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; and when taken in the oral cavity, the medicinal composition disintegrates in 30 seconds.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; and the medicinal composition has a hardness of 2 kp or higher.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; and a total of respective contents of the two or more sugar alcohols and/or saccharides is from 75.0 to 99.95% by weight based on a whole weight of the medicinal composition.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; and a content of one having a lowest melting point of the two or more sugar alcohols and/or saccharides is from 0.3 to 50.0% by weight based on a total of respective content(s) of the remaining one(s) of the two or more sugar alcohols and/or saccharides.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; the two or more sugar alcohols and/or saccharides comprise erythritol and trehalose; and a content of trehalose is from 0.3 to 50.0% by weight based on a content of erythritol.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; the two or more sugar alcohols and/or saccharides comprise erythritol and one of xylitol and sorbitol; and a content of one of xylitol and sorbitol is from 0.3 to 50.0% by weight based on a content of erythritol.

The present invention also relates to a medicinal composition produced by at least mixing, compression and heating, wherein the medicinal composition comprises two or more of sugar alcohols and/or saccharides, and an active ingredient; a difference between a melting point of one having a highest content of the two or more sugar alcohols and/or saccharides and that of any remaining one of the two or more sugar alcohols and/or saccharides is 5°C or greater; the two or more sugar alcohols and/or saccharides comprise mannitol and one of xylitol, sorbitol and treharose; and a content of any one of xylitol, sorbitol and trehalose is from 0.3 to 50.0% by weight based on a content of mannitol.

The present invention also relates to a process for the production of a medicinal composition, comprising steps of preparing a mixture of an active ingredient and two or more of sugar alcohols and/or saccharides, compressing the mixture into a compact and heating the compact, characterized in that a difference between a melting point of one having a highest content of the two or more sugar alcohols and/or saccharides and that of any remaining one of the two or more sugar alcohols and/or saccharides is 5°C or greater.

### Best Modes for Carrying out the Invention

The medicinal compositions according to the present invention are each characterized in that it comprises two or more of sugar alcohols and/or saccharides and a difference between a melting point of one having a highest content of said two or more sugar alcohols and/or saccharides and that of any remaining one of said two or more sugar alcohols and/or saccharides is 5°C or greater. The medicinal compositions according to the present invention make use of sintering action of the sugar alcohol(s) and/or saccharide(s) the melting point(s) of which is(are) lower by 5°C or more than the one having the highest content of the two or more sugar alcohols and/or saccharides. The term "sintering action" as used herein means that by subjecting a compact of powder to heating treatment around a melting point of a low melting-point material contained in the compact, cohesion between powder particles and contraction and densification of the compact are induced, resulting in solidification or densification of the compact. In the present invention, the inter-particle strength can be enhanced by cooling a medicinal composition, which contains the low melting-point sugar alcohol(s) and/or saccharide(s), after heating the medicinal composition around the low melting point. In other words, the medicinal composition can be solidified or densified such that hardness can be imparted to extent sufficient to withstand actual use. Incidentally, the expression "hardness .... to extent sufficient to withstand actual use" means a hardness sufficient to withstand production, distribution and use. Especially when the medicinal composition is in the form of tablets, this expression means a degree of strength sufficient to withstand without breakage when packaged with a PTP (press through package) sheet or taken out of the package. In the case of a tablet, the hardness sufficient to withstand actual use differs depending on its size and shape. However, its preferred illustrative hardness may be 0.5 kp or higher when its diameter or maximum length is less than 8 mm, 1 kp or higher when its diameter or maximum length is 8 mm or more but less than 10 mm, 2 kp or higher when its diameter or maximum length is 10 mm or more but less than 15 mm, 3 kp or higher when its diameter or maximum length is 15 mm or more but less than 20 mm, or 4 kp or higher when its diameter or maximum length is 20 mm or more.

Usable examples of the sugar alcohol(s) and/or saccharide(s) contained in each medicinal composition of the present invention can include monosaccharides, disaccharides and sugar alcohols. Specific examples can include erythritol, mannitol, lactitol, lactose, glucose, sucrose, maltitol, xylitol, sorbitol, trehalose and fructose. Any two of these saccharides and/or sugar alcohols can be used, or three or more of them can be used in combination. It is, however, preferred that the difference between the melting point of one having a highest content of the contained sugar alcohol(s) and/or saccharide(s) and that of any remaining one of the sugar alcohol(s) and/or saccharide(s) is 5°C or greater.

For the contents of the two or more sugar alcohols and/or saccharides, their suitable ranges are determined depending upon the combination of the contained sugar alcohol(s) and/or saccharide(s) and the production conditions. A specific description will hereinafter be made about the contents of the sugar alcohol(s) and/or saccharide(s) in each medicinal composition according to the present invention. It is to be noted that the contents to be described next were each calculated by excluding the active ingredient and ingredients other than the sugar alcohol(s) and/or saccharide(s), such as additives which may be added as needed. The content of one having the lowest melting point of the individual sugar alcohol(s) and saccharide(s) contained may preferably be from 0.1 to 75.0% by weight based on the total of the contents of the remaining sugar alcohol(s) and saccharide(s), with a range of from 0.3 to 50.0% by weight being particularly preferred.

In the case of a combination of erythritol and trehalose, the content of trehalose may preferably be from 0.1 to 75.0% by weight based on the content of erythritol, with a range of from 0.3 to 50.0% being particularly preferred. In the case of a combination of erythritol with one of xylitol and sorbitol, the content of one of xylitol and sorbitol may preferably be from 0.1 to 75.0% by weight based on the content of erythritol, with a range of from 0.3 to 50.0% by weight being particularly preferred. In the case of a combination of mannitol and one of xylitol, sorbitol and trehalose, the content of any one of xylitol, sorbitol and trehalose may preferably be from 0.1 to 75.0% by weight based on the content of mannitol, with a range of from 0.3 to 50.0% by weight being particularly preferred. In the case of a combination of lactose and one of xylitol, sorbitol and trehalose, the content of one of xylitol, sorbitol and trehalose may preferably be from 0.1 to 75.0% by weight based on the content of lactose, with a range of from 0.3 to 50.0% by weight being particularly preferred.

The medicinal compositions according to the present invention can bring about the advantageous effects of the present invention even when they contain one or more additives other than the above-described sugar alcohols and/or saccharides. Illustrative of the additives are lubricants, disintegrants, diluents, binders, colorants, flavoring agents, sweeteners, effervescents, surfactants and glidants.

No particular limitation is imposed on the active ingredient for use in the present invention insofar as it can be administered orally. Usable examples can include one or more active ingredients selected from vitamins, antipyretic-analgesic-antiphlogistic drugs, antihistamines, antitussives, gastric mucosa healing or repairing agents, analgesic-antispasmodic agents, antipsychotics, antiemetics, antidepressants, H₁ receptor antagonists, chemotherapeutics, antibiotics, depressors, antiarrhythmics, antianxiety agents, and ACE inhibitors. Each medicinal composition according to the present invention may contain the active ingredient generally in a proportion of from 0.05 to 75% by weight, preferably in a proportion of from 0.05 to 50% by weight, more preferably in a proportion of from 0.05 to 25% by weight.

Specific examples of these active ingredients can include thiamine hydrochloride, nicotinamide, aspirin, acetaminophen, indomethacin, diphenhydramine hydrochloride, procaterol hydrochloride, meclofenoxate hydrochloride, lorazepam, phenobarbital, timiperone, calcium p-aminosalicylate, ampicillin, carmofur, captopril, nifedipine, procainamide hydrochloride, perindopril erbumine, alimemazine tartrate, lofepramine hydrochloride, isoniazid, baclofen, cetirizine hydrochloride, isoxsuprine hydrochloride, N-methylscopolamine methylsulfate, trihexylphenidyl hydrochloride, timiperone, and oxypertine.

Further, the medicinal compositions according to the present invention can be produced without using water. Water-unstable active ingredients are, therefore, suited especially for medicinal compositions according to the present invention. The term "water-unstable active ingredient" as used herein means that under storage conditions of 25°C, 75%RH and 3 months, its content decreases by 5% or more compared with the initial value. Illustrative are thiamine hydrochloride, nicotinamide, aspirin, acetaminophen, indomethacin, diphenhydramine hydrochloride, procaterol hydrochloride, meclofenoxate hydrochloride, lorazepam, phenobarbital, calcium p-aminosalicylate, ampicillin, carmofur, captopril, nifedipine, procainamide hydrochloride, and perindopril erbumine.

As each medicinal composition of the present invention is obtained by heating, thermally-stable active ingredients are suited for the present invention. The term "thermally-stable active ingredient" as used herein means an active ingredient whose melting point or decomposition point is 140°C or higher. Illustrative are alimemazine tartrate, lofepramine hydrochloride, isoniazid, baclofen, cetirizine hydrochloride, isoxsuprine hydrochloride, N-methylscopolamine methylsulfate, trihexylphenidyl hydrochloride, timiperone, oxypertine, and perindopril erbumine.

The medicinal composition according to the present invention can be produced by preparing a mixture of the active ingredient, the two or more of sugar alcohols and/or saccharides and one or more additives, which may be added as needed, subjecting the mixture to compression, and then subjecting the thus-compressed compacts to heating.

A description will next be made of an example of the process of the present invention for the production of the medicinal composition.

The production of the medicinal composition according to the present invention can be conducted by a commonly-used, medicinal preparation manufacturing machine.

The active ingredient and the two sugar alcohol(s) and/or saccharide(s) are mixed together using a v-blender, a double cone blender, a fluidized-bed granulating dryer, a wet shear granulator, a Nauta mixer or the like.

The resulting mixture is compressed into compacts by a conventional tabletting machine such as a single-punch tableting machine or a rotary tableting machine. The compression pressure upon tableting can be set depending on the hardness of the compacts and their disintegration or dissolution property when it is taken in the oral cavity. Accordingly, the compression pressure may be from 100 to 2,000 kgf or so, preferably from 200 to 1,800 kgf or so, more preferably from 300 to 1,500 kgf or so.

By a conventionally-employed dryer, for example, an air-convection constant-temperature oven, constant-temperature drying oven, vacuum drying oven or microwave oven, the compacts are then heated to obtain the medicinal composition of the present invention. The heating temperature may be from 60 to 180°C, preferably from 70 to 160°C, more preferably from 80 to 140°C. The heating time may be from 0.5 to 240 minutes, preferably from 1 to 120 minutes, more preferably from 3 to 90 minutes.

In the mixing step, one or more additives may be added as needed. The production process according to the present invention can be practiced with additional incorporation of a granulating step subsequent to the mixing step. The granulating step can be conducted using a fluidized-bed granulating dryer, a wet shear granulator, a tumbling or fluidized-bed granulator, a tumble granulator, an extrusion granulator or the like.

Incidentally, granulation includes wet granulation and dry granulation. The term "wet granulation" as used herein means that wet granulation is conducted using a solution or suspension, which has been prepared by adding and dissolving or suspending one or more additives to the above-described sugar alcohols and/or saccharides as needed, or water and conducting wet granulation. Specific processes of wet granulation include (1) fluidized-bed granulation process, which comprises forming a fluidized bed of the above-described ingredients by means of an air stream, and spraying a solution or suspension, which has been prepared by adding and dissolving or suspending one or more additives to the above-described sugar alcohols and/or saccharides as needed, or water into the fluidized bed while effecting drying such that particles are caused to cohere and agglomerate into granules by liquid linking; (2) agitating granulation process, which comprises adding a solution, which has been prepared by dissolving and/or suspending sugar alcohols and/or saccharides and optionally, one or more additives, or water to the above-described ingredient and conducting granulation under agitation; (3) high-speed agitating granulation process similar to the agitating granulation except for application of high shear force, which comprises conducting granulation by adding a solution, which has been prepared by dissolving and/or suspending sugar alcohols and/or saccharides and optionally, one or more additives, or water while agitating the above-described ingredient at a high speed; (4) extrusion granulating process, which comprises adding water or the like to the above-described ingredients, conducting kneading, and pressing the thus-kneaded mass against a die or screen such that the kneaded mass is extruded into granules; and (5) rolling granulation process, which comprises spraying or coating a solution, which has been prepared by dissolving and/or suspending sugar alcohols and/or saccharides and optionally, one or more additives, or water onto the above-described ingredients, which are rolling, such that spherical particles are formed [see "Iyakuhin no Kaihatsu (Developments of Medicines)", Volume 11 - "Seizai no Tan-i Sosa and Kikai (Unit Operations and Machines for Pharmaceutical Preparations), Hirokawa Publishing Company]. These processes are all usable in the present invention.

The concentration of the solution or suspension, which has been prepared by optionally adding one or more additives to the sugar alcohols and/or polysaccharides and dissolving or suspending them, may range preferably from 5 to 80 w/w %, more preferably from 10 to 70 w/w %, still more preferably from 20 to 60 w/w %.

The "above-described ingredients" in the above-described processes (1) to (5) can be either the entire ingredients, that is, the sugar alcohols and/or saccharides, the active ingredient and one or more additives which may be added as needed or some of the ingredients (with the proviso that the sugar alcohols and/or polysaccharides are essential).

Further, when an active ingredient is unstable with water, it is possible to mix the active ingredient after drying, which is conducted subsequent to a granulation operation, as needed, without including the active ingredient in the above-described ingredients.

The term "dry granulation" as used herein means that the above-described ingredients are granulated by compressing and forming the ingredients as are, and then grinding them into a suitable size.

The medicinal compositions according to the present invention are characterized by a shorter wetting time as measured by a wetting test already known from a technical paper [Y. Bi, H. Sunada, Y. Yonezawa, K. Danjo, A. Otsuka and K. Iida, Chem. Pharm. Bull., **44**(11), 2121-2127 (1996)]. The wetting time may be generally 60 seconds or shorter, preferably 30 seconds or shorter, more preferably 10 seconds or shorter.

The medicinal compositions according to the present invention are characterized in that they rapidly disintegrate in the oral cavity. The term "intraoral disintegration time" as used herein means a time required until a tablet completely disintegrates or dissolves out of the oral cavity when a healthy male adult takes it in his oral cavity without water. The intraoral disintegration time may be generally 90 seconds or shorter, preferably 60 seconds or shorter, more preferably 30 seconds or shorter, still more preferably 15 seconds or shorter. When taken in a usual manner, a tablet can disintegrate in a still shorter time by the taker's licking of the tablet or by the taker's compression of the tablet between his or her tongue and mouth roof. Medicinal compositions the intraoral disintegration time of each of which is 15 seconds or shorter are, therefore, particularly preferred as they can be considered to have sufficient intraoral disintegration property from the viewpoint of ease in use upon being taken by those aged, infants, patients restricted in water intake, or like people.

It has been reported that a good correlation is shown between an intraoral disintegration time and a wetting time as measured by a wetting test [Y. Bi, H. Sunada, Y. Yonezawa, K. Danjo, A. Otsuka and K. Iida, Chem. Pharm. Bull., **44**(11), 2121-2127 (1996)].

More preferred in the present invention are medicinal compositions each of which contains an active ingredient such that the content of the active ingredient falls within a range of from 0.05 to 25 wt.% based on a whole amount of the medicinal composition, has a wetting time of 10 seconds or shorter when tested by a wetting test, disintegrates in 30 seconds or shorter when taken in the oral cavity, and has a hardness of 2 kp or higher.

### Examples

The present invention will hereinafter be described in further detail based on the following Examples. It should however be borne in mind that the present invention shall not be limited to or by the Examples.

### Testing methods

To more specifically demonstrate the advantageous effects of the present invention, the tables obtained in the following Comparative Examples and Examples were tested for the following properties of preparations.

### (Hardness test)

Using a tablet hardness tester manufactured by Elbaker GmbH, the breaking strength of each tablet in the direction of its diameter was measured.

### (Intraoral disintegration test)

Each tablet was taken without water in the oral cavity of a healthy male adult, and the time required until the tablet completely disintegrated or dissolved out of his oral cavity was measured.

### (Wetting test - Y. Bi, H. Sunada, Y. Yonezawa, K. Danjo, A. Otsuka and K. Iida, Chem. Pharm. Bull., 44(11), 2121-2127, 1996)

A sheet of commercial tissue paper was folded into 10 cm long x 11 cm wide and was placed in a Petri dish made of plastics. The folded tissue paper was moistened with water (6 mL). A tablet was placed on the moistened tissue paper, and the time (wetting time) required until water reached an upper surface of the tablet was measured.

This wetting test is performed when the above-described intraoral disintegration test is not applied due to properties of an active ingredient.

### Example 1

Erythritol and trehalose were mixed at a ratio of 299:1, and using an autograph (manufactured by Shimadzu Corporation), single-punch tableting of the resulting mixture was conducted (weight: 300 mg, compression pressure: 500 kgf, punch: 10 mm in diameter). The thus-obtained compacts were heated at 120°C for 15 minutes, and were then allowed to cool down at room temperature to obtain intraoral rapid-disintegrating tablets.

### Example 2

Erythritol and trehalose were mixed at a ratio of 29.5:0.5, and single-punch tableting was conducted in a similar manner as in Example 1. The resulting compacts were heated at 95°C for 60 minutes, and were then allowed to cool down at room temperature to obtain intraoral rapid-disintegrating tablets.

### Example 3

Erythritol and trehalose were mixed at a ratio of 29:1, and single-punch tableting was conducted in a similar manner as in Example 1. The resulting compacts were heated at 95°C for 60 minutes, and were then allowed to cool down at room temperature to obtain intraoral rapid-disintegrating tablets.

### Example 4

Erythritol and trehalose were mixed at a ratio of 2:1, and single-punch tableting was conducted in a similar manner as in Example 1. The resulting compacts were heated at 80°C for 90 minutes, and were then allowed to cool down at room temperature to obtain intraoral rapid-disintegrating tablets.

### Comparative Example 1

Based on the formula shown in Table 1, single-punch tableting was conducted in a similar manner as in Example 1. The resulting compacts were heated at 95°C for 60 minutes, and were then allowed to cool down at room temperature to obtain samples for comparison.

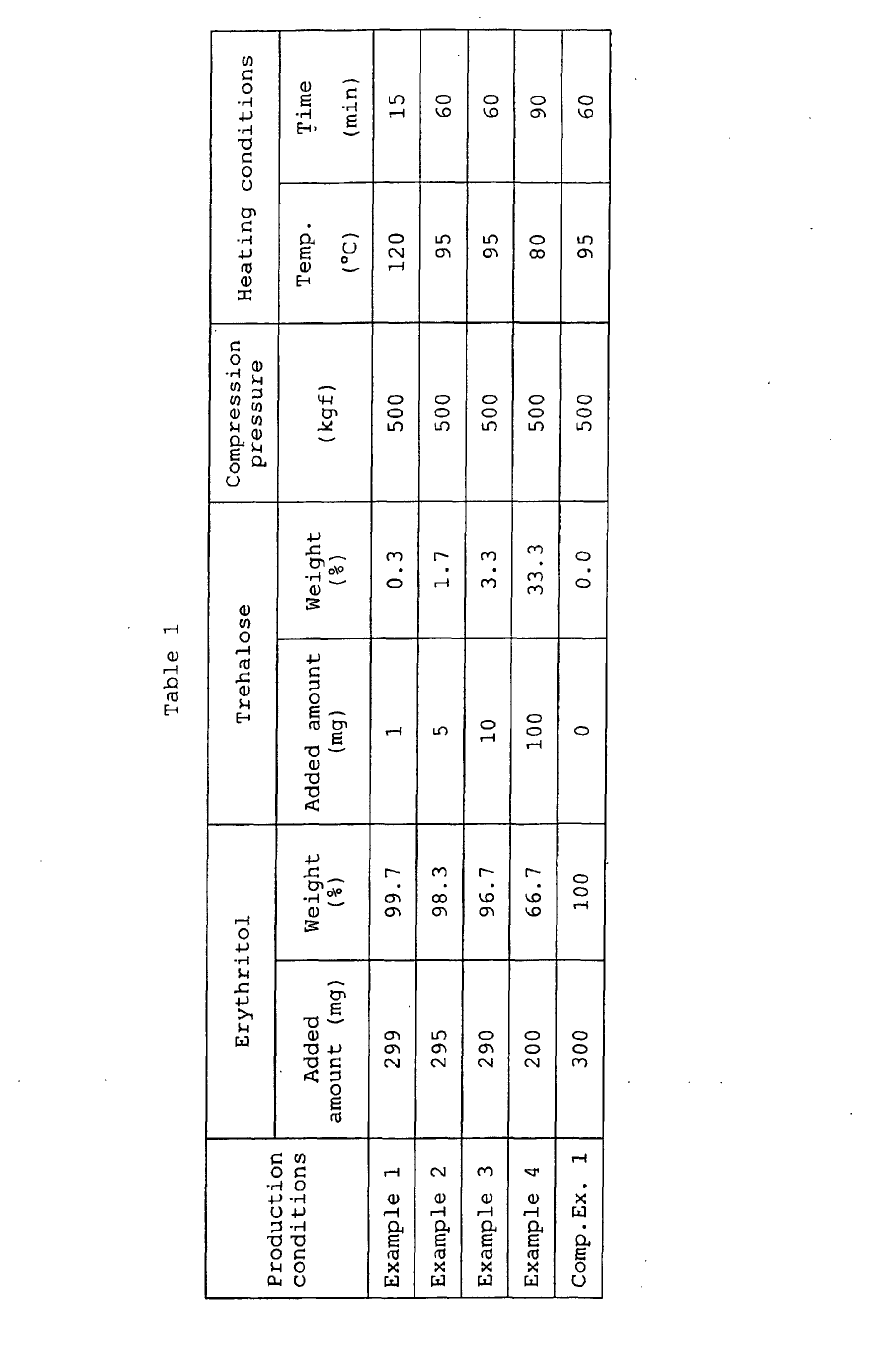

The hardness and intraoral disintegration time of each tablet after its heating was measured. The results are presented in Table 2.

**Table 2**

| Production conditions | Hardness (kp) | Intraoral disintegration time (sec) |
|---|---|---|
| Example 1 | 4.3 | 8 |
| Example 2 | 2.0 | 5 |
| Example 3 | 2.8 | 10 |
| Example 4 | 2.0 | 12 |
| Comp. Ex. 1 | 0.8 | 8 |

It has been confirmed that tablets, which disintegrate in a time as short as 30 seconds in the oral cavity and have a practical hardness of 2 kp or higher, can be obtained by adding trehalose, a low melting-point sugar alcohol, to erythritol and heating the resulting mixture subsequent to compression (Examples 1-4). In Comparative Example 1 in which trehalose, a low melting-point sugar alcohol, was not added, on the other hand, it was ascertained that, although the intraoral disintegration time was short, the hardness was as low as 0.8 kp and did not permit providing the tablets for actual use.

### Example 5

Erythritol and xylitol were mixed at a ratio of 29:1, and using an autograph (manufactured by Shimadzu Corporation), single-punch tableting of the resulting mixture was conducted (weight: 300 mg, compression pressure: 500 kgf, punch: 10 mm in diameter). The thus-obtained compacts were heated at 90°C for 60 minutes, and were then allowed to cool down at room temperature to obtain intraoral rapid-disintegration tablets.

### Example 6

Intraoral rapid-disintegrating tablets were obtained by conducting a similar procedure as in Example 5 except that xylitol was changed to sorbitol.

### Example 7

Single-punch tableting was conducted by a similar procedure as in Example 5 except that erythritol and xylitol were changed to mannitol and trehalose, respectively, and mannitol and trehalose were mixed at a weight ratio of 11:1. The thus-obtained compacts were heated at 120°C for 60 minutes, and were then allowed to cool down at room temperature to obtain intraoral rapid-disintegrating tablets.

### Example 8

Single-punch tableting was conducted by a similar procedure as in Example 7 except that trehalose was changed to xylitol. The thus-obtained compacts were heated at 90°C for 60 minutes, and were then allowed to cool down at room temperature to obtain intraoral rapid-disintegrating tablets.

### Example 9

Single-punch tableting was conducted by a similar procedure as in Example 8 except that xylitol was changed to sorbitol. The thus-obtained compacts were heated at 110°C for 60 minutes, and were then allowed to cool down at room temperature to obtain intraoral rapid-disintegrating tablets.

### Example 10

Single-punch tableting was conducted by a similar procedure as in Example 9 except that mannitol and sorbitol were changed to lactose and trehalose, respectively. The thus-obtained compacts were heated at 160° for 60 minutes, and were then allowed to cool down at room temperature to obtain intraoral rapid-disintegrating tablets.

### Example 11

Single-punch tableting was conducted by a similar procedure as in Example 10 except that trehalose was changed to xylitol. The thus-obtained compacts were heated at 100°C for 15 minutes, and were then allowed to cool down at room temperature to obtain intraoral rapid-disintegrating tablets.

### Example 12

Single-punch tableting was conducted by a similar procedure as in Example 11 except that xylitol was changed to sorbitol. The thus-obtained compacts were heated at 110°C for 5 minutes, and were then allowed to cool down at room temperature to obtain intraoral rapid-disintegrating tablets.

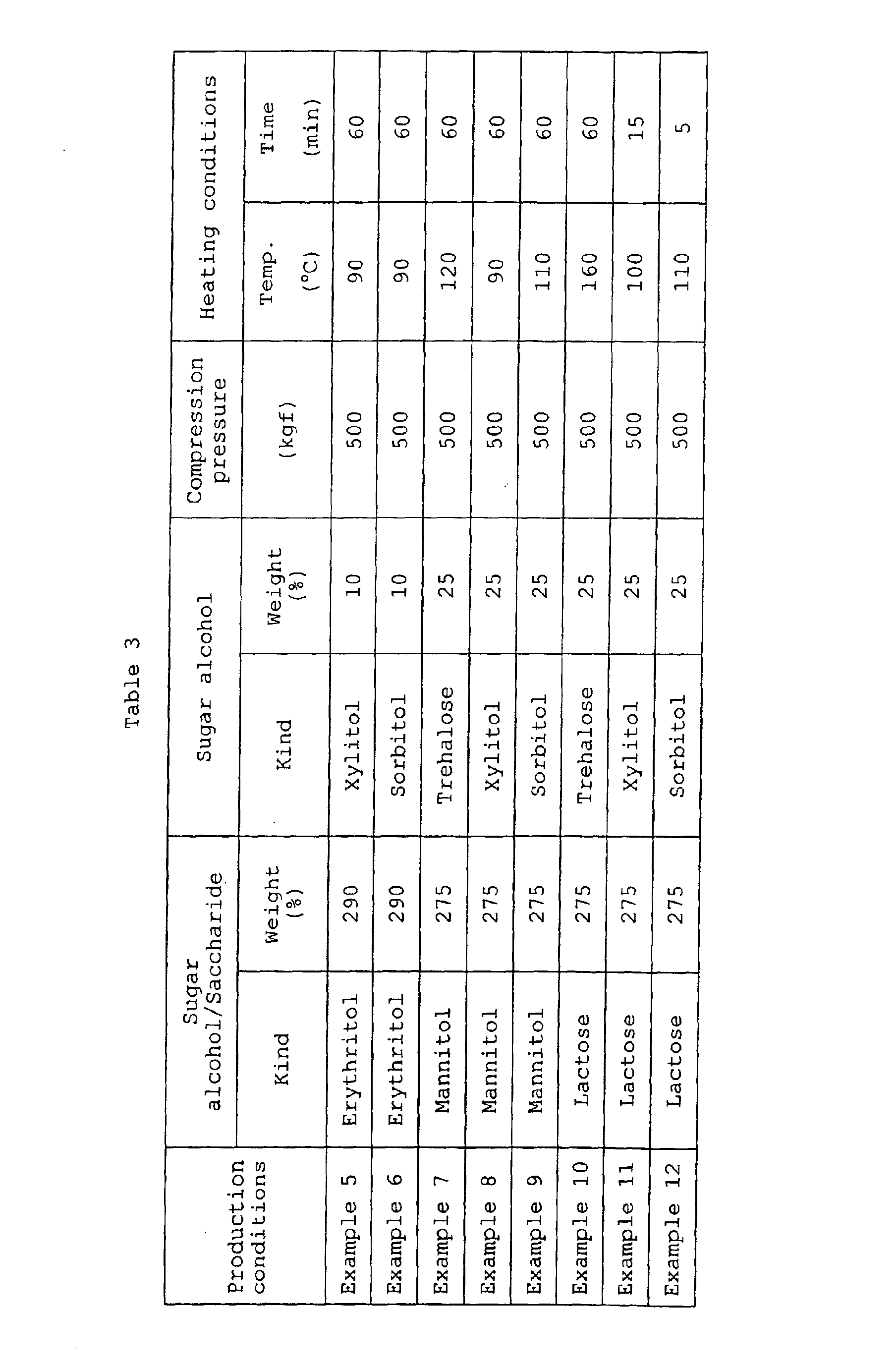

The hardness and intraoral disintegration time of each tablet after its heating were measured. The results are presented in Table 4.

**Table 4**

| | Hardness (kp) | | Intraoral disintegration time (sec) |
|---|---|---|---|
| | Before heating | After heating | |
| Example 5 | 0.20 | 3.1 | 15 |
| Example 6 | 0.71 | 2.5 | 9 |
| Example 7 | 1.02 | 3.0 | 25 |
| Example 8 | - | 2.8 | 18 |
| Example 9 | 1.63 | 3.6 | 22 |
| Example 10 | - | 3.1 | 47 |
| Example 11 | - | 2.5 | 37 |
| Example 12 | 1.43 | 2.6 | 22 |

It has been confirmed that in addition to the combinations of erythritol and trehalose, the combination of erythritol and xylitol, the combination of erythritol and sorbitol, the combination of mannitol and trehalose, the combination of mannitol and xylitol, the combination of mannitol and sorbitol and the combination of lactose and sorbitol can each achieve a short intraoral integration time not longer than 30 seconds and a practical hardness of 2 kp or higher.

### Example 13

Erythritol and trehalose were mixed at a ratio of 29:1, and using an autograph (manufactured by Shimadzu Corporation), single-punch tableting of the resulting mixture was conducted (weight: 300 mg, compression pressure: 100 kgf, punch: 10 mm in diameter). The thus-obtained compacts were heated at 95°C for 60 minutes, and were then allowed to cool down at room temperature to obtain intraoral rapid-disintegrating tablets.

### Example 14

Erythritol and trehalose were mixed at a ratio of 29:1, and single-punch tableting was conducted (weight: 300 mg, compression pressure: 300 kgf, punch: 10 mm in diameter). Following the procedure of Example 13, intraoral rapid-disintegrating tablets were then obtained.

### Example 15

Erythritol and trehalose were mixed at a ratio of 29:1, and single-punch tableting was conducted (weight: 300 mg, compression pressure: 500 kgf, punch: 10 mm in diameter). Following the procedure of Example 13, intraoral rapid-disintegrating tablets were then obtained.

### Example 16

Erythritol and trehalose were mixed at a ratio of 29:1, and single-punch tableting was conducted (weight: 300 mg, compression pressure: 800 kgf, punch: 10 mm in diameter). Following the procedure of Example 13, intraoral rapid-disintegrating tablets were then obtained.

### Example 17

Erythritol and trehalose were mixed at a ratio of 29:1, and single-punch tableting was conducted (weight: 300 mg, compression pressure: 1,000 kgf, punch: 10 mm in diameter). Following the procedure of Example 13, intraoral rapid-disintegrating tablets were then obtained.

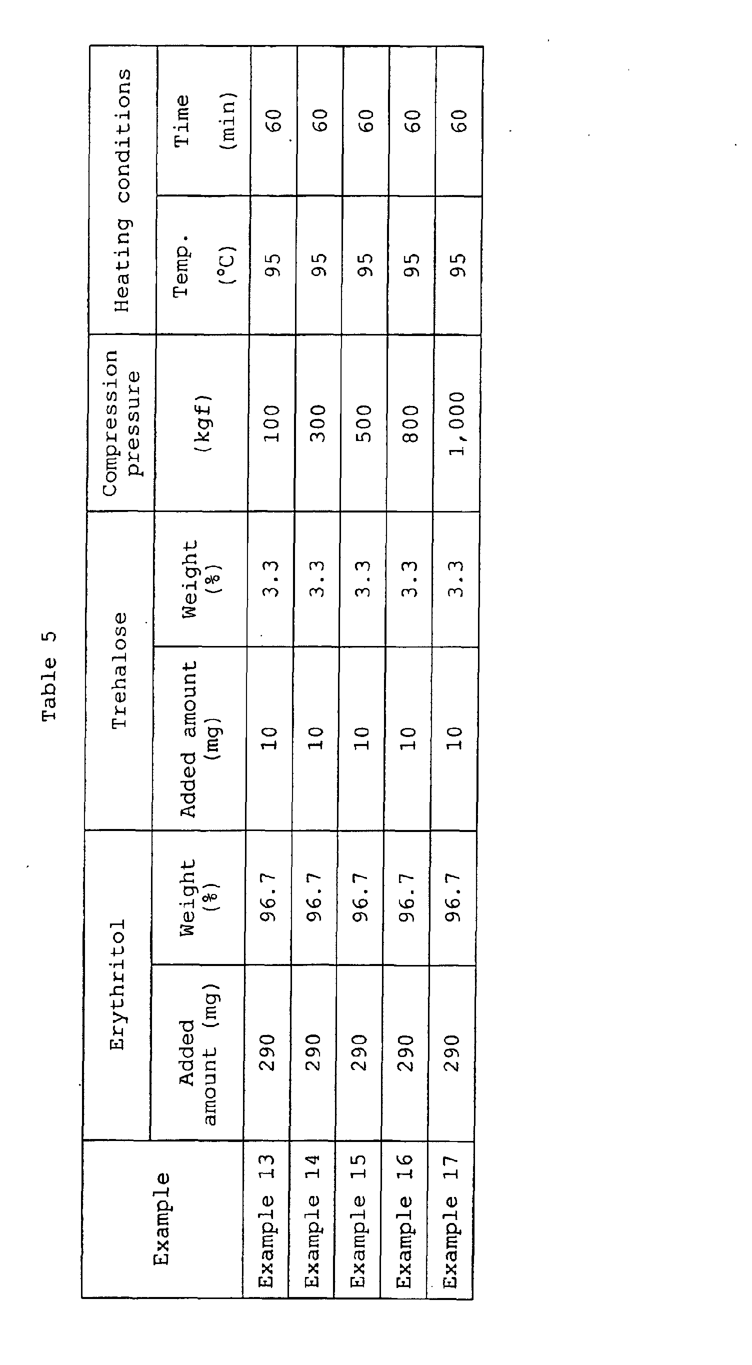

The hardness and intraoral disintegration time of each tablet after its heating were measured. The results are presented in Table 6.

**Table 6**

| Example | Hardness (kp) | Intraoral disintegration time (sec) |
|---|---|---|
| Example 13 | 1.4 | 5 |
| Example 14 | 2.3 | 7 |
| Example 15 | 2.8 | 10 |
| Example 16 | 2.8 | 11 |
| Example 17 | 2.9 | 12 |

Since the use of 100 kgf as compression pressure in compression resulted in the low hardness of 1.4 kp even after the subsequent heating and failed to obtain any hardness sufficient for practical use, it has been confirmed that compression pressure of a certain level or higher is needed for compression. On the other hand, the compression pressures of 300 kgf and higher achieved hardness needed for practical use, that is, hardness of 2 kp or higher. However, the hardness remained unchanged when the compression pressure was increased from 500 kgf to 800 kgf. Even at the compression pressure of 1,000 kgf, the hardness increased by only 0.1 kp from that achieved at 500 kgf. It has, therefore, been ascertained that compression pressure of a certain level or higher is sufficient for compression and heating is indispensable for eventually achieving practical hardness.

### Example 18

Timiperone (1 mg) and trehalose (6 mg) were mixed with erythritol (193 mg), and using an autograph (manufactured by Shimadzu Corporation), single-punch tableting of the resulting mixture was conducted (weight: 200 mg, compression pressure: 500 kgf, punch: 8 mm in diameter). The thus-obtained compacts were heated at 120°C for 6 minutes, and were then allowed to cool down at room temperature to obtain intraoral rapid-disintegrating tablets.

### Comparative Example 2

Based on the formula shown in Table 7, samples for comparison were obtained in a similar manner as in Example 18.

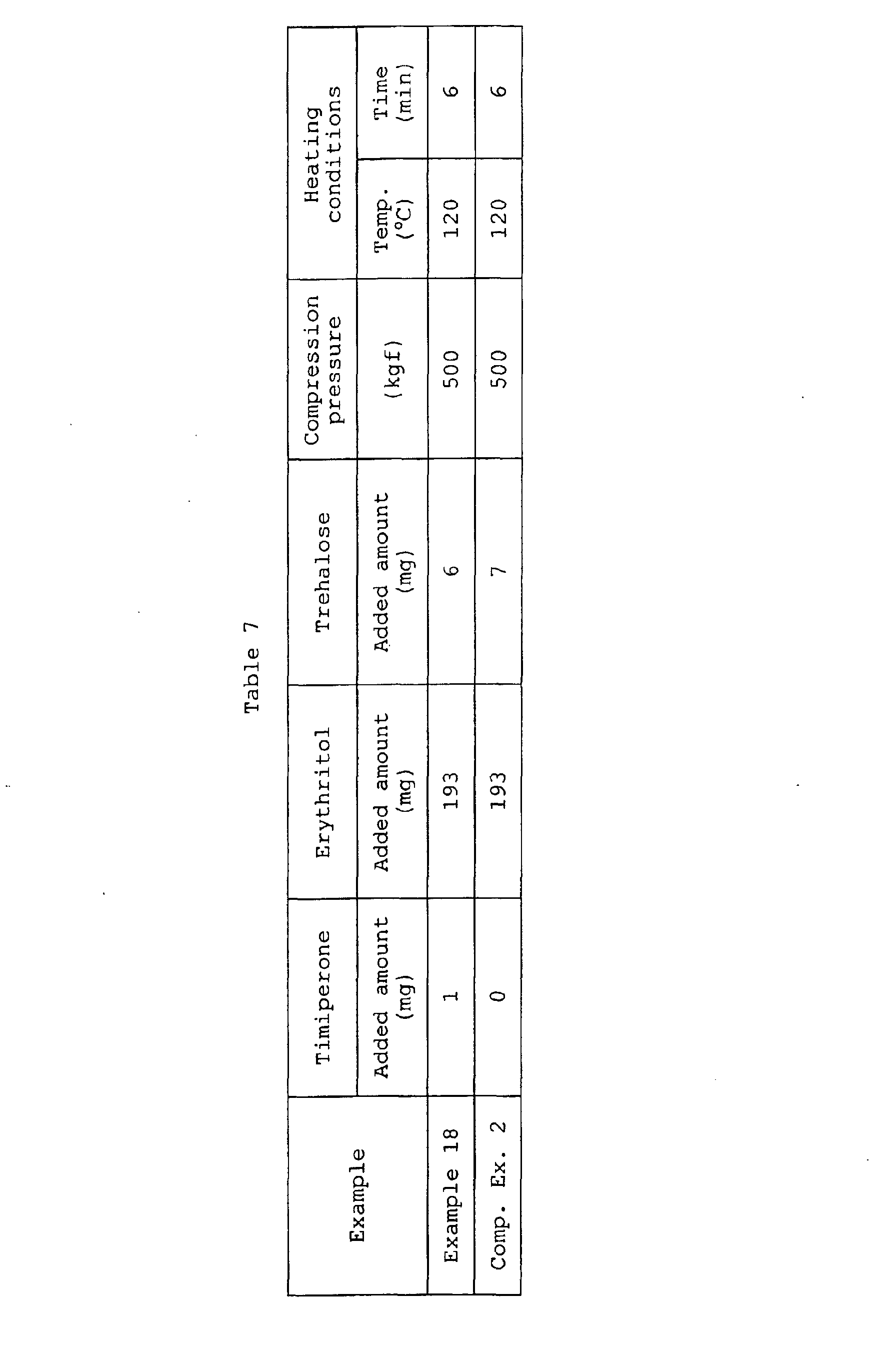

The hardness, intraoral disintegration time and wetting time of each tablet after its heating were measured. The results are presented in Table 8.

**Table 8**

| Example | Hardness (kp) | Intraoral disintegration time (sec) | Wetting time (sec) |
|---|---|---|---|
| Example 18 | 3.5 | - | 4 |
| Comp. Ex. 2 | 3.2 | 13 | 4 |

No difference was observed in wetting time between Example 18 and Comparative Example 5. As an intraoral disintegration time is known to show a good correlation with a wetting time, the tablets of Example 18 have been confirmed to have similar rapid intraoral disintegrating property as those of Comparative Example 2.

### Example 19

Perindopril erbumine (1 mg) and trehalose (6 mg) were mixed with erythritol (193 mg), and using an autograph (manufactured by Shimadzu Corporation), single-punch tableting of the resulting mixture was conducted (weight: 200 mg, compression pressure: 500 kgf, punch: 8 mm in diameter). The thus-obtained compacts were heated at 120°C for 6 minutes, and were then allowed to cool down at room temperature to obtain intraoral rapid-disintegrating tablets.

### Comparative Example 3

Based on the formula shown in Table 9, samples for comparison were obtained in a similar manner as in Example 19.

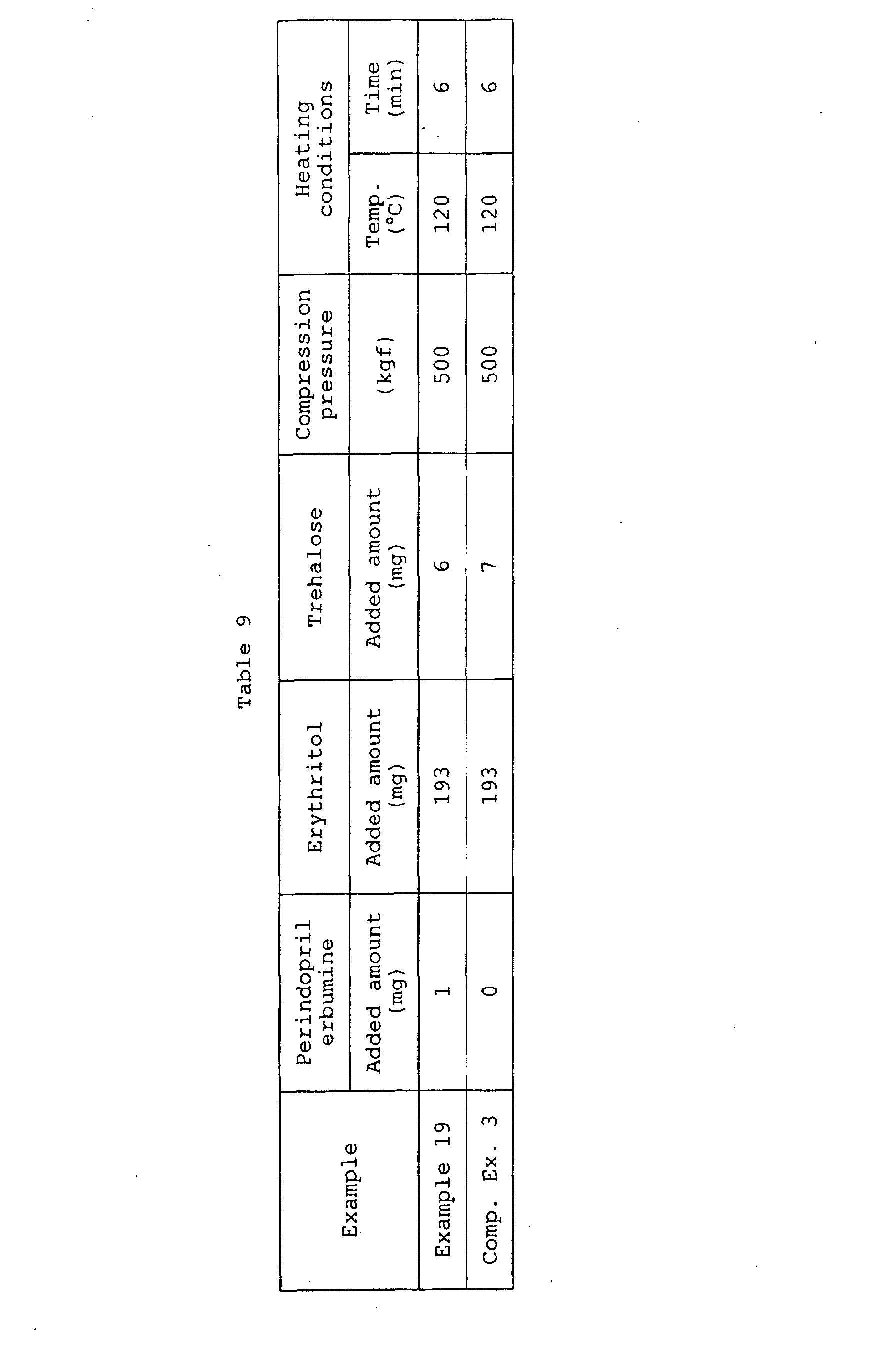

The hardness, intraoral disintegration time and wetting time of each tablet after its heating were measured. The results are presented in Table 10.

**Table 10**

| Example Example | Hardness (kp) | Intraoral disintegration time (sec) | Wetting Time (sec) |
|---|---|---|---|
| Example 19 | 4.4 | - | 4 |
| Comp. Ex. 3 | 3.2 | 13 | 4 |

No difference was observed in wetting time between Example 19 and Comparative Example 3. As an intraoral disintegration time has a good correlation with a wetting time, the tablets of Example 19 have been confirmed to have similar rapid intraoral disintegration property as those of Comparative Example 3.

### Example 20

In a fluidized-bed granulating dryer ("FLOW COATER Mini", manufactured by Freund Industrial Co., Ltd.), erythritol (190 g) was granulated with a 26.7 w/w% aqueous solution (37.5 mL) of xylitol at a atomizing air pressure of 1.5 kg/cm² and a feed rate of 0.8 mL/min. Subsequent to drying, single-punch tableting of the resulting powder was conducted using an autograph (manufactured by Shimadzu Corporation)(weight: 300 mg, compression pressure: 500 kgf, punch: 10 mm in diameter). The thus-obtained compacts were heated at 90°C for 15 minutes, and were then allowed to cool down at room temperature to obtain intraoral rapid-disintegrating tablets.

### Example 21

Granulation and single-punch tableting were conducted by a similar procedure as in Example 20 except that erythritol was changed to mannitol. The thus-obtained compacts were heated at 90°C for 15 minutes, and were then allowed to cool down at room temperature to obtain intraoral rapid-disintegrating tablets.

### Example 22

Granulation and single-punch tableting were conducted by a similar procedure as in Example 20 except that erythritol was changed to lactose. The thus-obtained compacts were heated at 90°C for 15 minutes, and were then allowed to cool down at room temperature to obtain intraoral rapid-disintegrating tablets.

### Example 23

In a fluidized-bed granulating dryer ("FLOW COATER Mini", manufactured by Freund Industrial Co., Ltd.), erythritol (190 g) was granulated with a 26.7 w/w% aqueous solution (37.5 mL) of trehalose at a atomizing air pressure of 1.5 kg/cm² and a feed rate of 0.8 mL/min. Subsequent to drying, single-punch tableting of the resulting powder was conducted using an autograph (manufactured by Shimadzu Corporation)(weight: 300 mg, compression pressure: 500 kgf, punch: 10 mm in diameter). The thus-obtained compacts were heated at 95°C for 15 minutes, and were then allowed to cool down at room temperature to obtain intraoral rapid-disintegrating tablets.

### Example 24

Granulation and single-punch tableting were conducted by a similar procedure as in Example 23 except that erythritol was changed to mannitol. The thus-obtained compacts were heated at 95°C for 15 minutes, and were then allowed to cool down at room temperature to obtain intraoral rapid-disintegrating tablets.

### Example 25

Granulation and single-punch tableting were conducted by a similar procedure as in Example 23 except that erythritol was changed to lactose. The thus-obtained compacts were heated at 95°C for 15 minutes, and were then allowed to cool down at room temperature to obtain intraoral rapid-disintegrating tablets.

### Example 26

In a fluidized-bed granulating dryer ("FLOW COATER Mini", manufactured by Freund Industrial Co., Ltd.), erythritol (190 g) was granulated with a 26.7 w/w% aqueous solution (37.5 mL) of sorbitol at a atomizing air pressure of 1.5 kg/cm² and a feed rate of 0.8 mL/min. Subsequent to drying, single-punch tableting of the resulting powder was conducted using an autograph (manufactured by Shimadzu Corporation)(weight: 300 mg, compression pressure: 500 kgf, punch: 10 mm in diameter). The thus-obtained compacts were heated at 100°C for 15 minutes, and were then allowed to cool down at room temperature to obtain intraoral rapid-disintegrating tablets.

### Example 27

Granulation and single-punch tableting were conducted by a similar procedure as in Example 26 except that erythritol was changed to mannitol. The thus-obtained compacts were heated at 100°C for 15 minutes, and were then allowed to cool down at room temperature to obtain intraoral rapid-disintegrating tablets.

### Example 28

Granulation and single-punch tableting were conducted by a similar procedure as in Example 26 except that erythritol was changed to lactose. The thus-obtained compacts were heated at 100°C for 15 minutes, and were then allowed to cool down at room temperature to obtain intraoral rapid-disintegrating tablets.

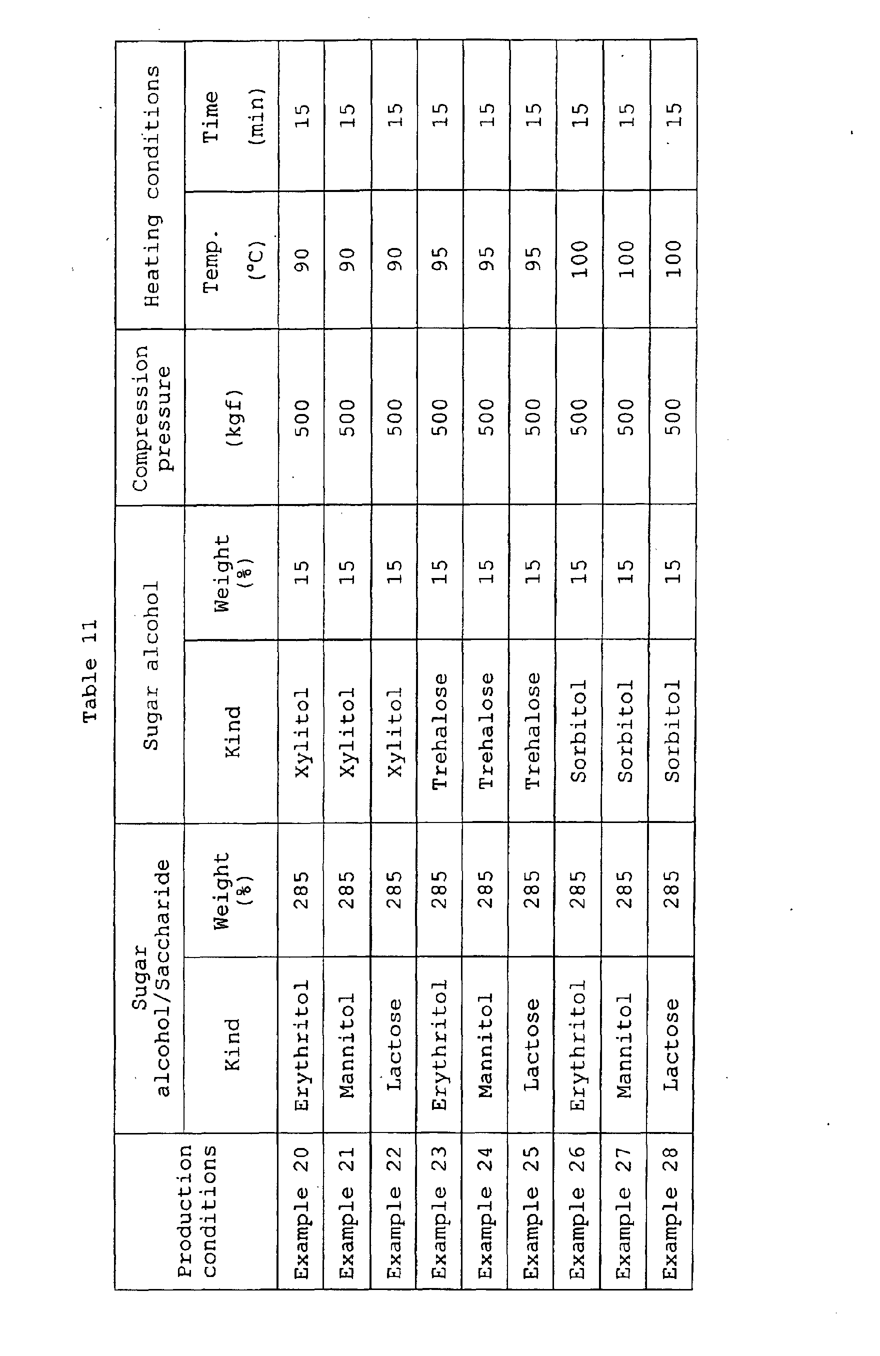

The hardness and intraoral disintegration time of each tablet after its heating were measured. The results are presented in Table 12.

**Table 12**

| Production conditions | Hardness (kp) | Intraoral disintegration time (sec) |
|---|---|---|
| Example 20 | 5.4 | 26 |
| Example 21 | 3.6 | 17 |
| Example 22 | 7.9 | 18 |
| Example 23 | 6.3 | 21 |
| Example 24 | 5.6 | 32 |
| Example 25 | 8.2 | 24 |
| Example 26 | 1.7 | 14 |
| Example 27 | 2.6 | 20 |
| Example 28 | 3.7 | 34 |

## Claims

1. A medicinal composition produced by at least mixing, compression and heating, wherein said medicinal composition comprises two or more of sugar alcohols and/or saccharides, and an active ingredient; and a difference between a melting point of one having a highest content of said two or more sugar alcohols and/or saccharides and that of any remaining one of said two or more sugar alcohols and/or saccharides is 5°C or greater.

2. A medicinal composition according to claim 1, wherein said heating is effected at 80 to 140°C.

3. A medicinal composition according to claim 1 or 2, wherein said heating is effected for 3 to 90 minutes.

4. A medicinal composition according to any one of claims 1-3, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

5. A medicinal composition according to any one of claims 1-4, which is produced by at least mixing, granulation, compression and heating.

6. A medicinal composition produced by at least mixing, compression and heating, wherein said medicinal composition comprises two or more of sugar alcohols and/or saccharides, and a water-unstable active ingredient; and a difference between a melting point of one having a highest content of said two or more sugar alcohols and/or saccharides and that of any remaining one of said two or more sugar alcohols and/or saccharides is 5°C or greater.

7. A medicinal composition according to claim 6, wherein said water-unstable active ingredient is selected from the following group:
thiamine hydrochloride, nicotinamide, aspirin, acetaminophen, indomethacin, diphenhydramine hydrochloride, procaterol hydrochloride, meclofenoxate hydrochloride, lorazepam, phenobarbital, calcium p-aminosalicylate, ampicillin, carmofur, captopril, nifedipine, procainamide hydrochloride, and perindopril erbumine.

8. A medicinal composition according to claim 6, wherein said water-unstable active ingredient is perindopril erbumine.

9. A medicinal composition according to any one of claims 6-8, wherein said heating is effected at 80 to 140°C.

10. A medicinal composition according to any one of claims 6-9, wherein said heating is effected for 3 to 90 minutes.

11. A medicinal composition according to any one of claims 6-10, wherein said sugar alcohols and/or saccharides are tow or more sugar alcohols and/or saccharides selected from the following group:
erythritol, mannitol, lactitol, lactose, glucose, sucrose, maltitol, xylitol, sorbitol, trehalose, and fructose.

12. A medicinal composition according to any one of claims 6-11, which is produced by at least mixing, granulation, compression and heating.

13. A medicinal composition produced by at least mixing, compression and heating, wherein said medicinal composition comprises two or more of sugar alcohols and/or saccharides, and an active ingredient a melting point or decomposition point of which is 140°C or higher; and a difference between a melting point of one having a highest content of said two or more sugar alcohols and/or saccharides and that of any remaining one of said two or more sugar alcohols and/or saccharides is 5°C or greater.

14. A medicinal composition according to claim 13, wherein said active ingredient the melting point or decomposition point of which is 140°C or higher is selected from the following group:
alimemazine tartrate, lofepramine hydrochloride, isoniazid, baclofen, cetirizine hydrochloride, isoxsuprine hydrochloride, N-methylscopolamine methylsulfate, trihexylphenidyl hydrochloride, timiperone, and oxypertine.

15. A medicinal composition according to claim 13, wherein said active ingredient the melting point or decomposition point of which is 140°C or higher is timiperone.

16. A medicinal composition according to any one of claims 13-15, wherein said heating is effected at 80 to 140°C.

17. A medicinal composition according to any one of claims 13-16, wherein said heating is effected for 3 to 90 minutes.

18. A medicinal composition according to any one of claims 13-17, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

19. A medicinal composition according to any one of claims 13-18, which is produced by at least mixing, granulation, compression and heating.

20. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; and said medicinal composition has a wetting time of 10 seconds or shorter when tested by a wetting test.

21. A medicinal composition according to claim 20, wherein said heating is effected at 80 to 140°C.

22. A medicinal composition according to claim 20 or 21, wherein said heating is effected for 3 to 90 minutes.

23. A medicinal composition according to any one of claims 20-22, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

24. A medicinal composition according to any one of claims 20-23, which is produced by at least mixing, granulation, compression and heating.

25. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; and when taken in the oral cavity, said medicinal composition disintegrates in 30 seconds.

26. A medicinal composition according to claim 25, wherein said heating is effected at 80 to 140°C.

27. A medicinal composition according to claim 25 or 26, wherein said heating is effected for 3 to 90 minutes.

28. A medicinal composition according to any one of claims 25-27, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

29. A medicinal composition according to any one of claims 25-28, which is produced by at least mixing, granulation, compression and heating.

30. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; and said medicinal composition has a hardness of 2 kp or higher.

31. A medicinal composition according to claim 30, wherein said heating is effected at 80 to 140°C.

32. A medicinal composition according to claim 30 or 31, wherein said heating is effected for 3 to 90 minutes.

33. A medicinal composition according to any one of claims 30-32, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

34. A medicinal composition according to any one of claims 30-33, which is produced by at least mixing, granulation, compression and heating.

35. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; and a total of respective contents of said two or more sugar alcohols and/or saccharides is from 75.0 to 99.95% by weight based on a whole weight of said medicinal composition.

36. A medicinal composition according to claim 35, wherein said heating is effected at 80 to 140°C.

37. A medicinal composition according to claim 35 or 36, wherein said heating is effected for 3 to 90 minutes.

38. A medicinal composition according to any one of claims 35-37, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

39. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; and a content of one having a lowest melting point of said two or more sugar alcohols and/or saccharides is from 0.3 to 50.0% by weight based on a total of respective content(s) of the remaining one(s) of said two or more sugar alcohols and/or saccharides.

40. A medicinal composition according to claim 39, wherein said heating is effected at 80 to 140°C.

41. A medicinal composition according to claim 39 or 40, wherein said heating is effected for 3 to 90 minutes.

42. A medicinal composition according to any one of claims 39-41, wherein said sugar alcohols and/or saccharides are two or more sugar alcohols and/or saccharides selected from the following group:
erythritol, mannitol, lactitol, lactose, glucose, sucrose, maltitol, xylitol, sorbitol, trehalose, and fructose.

43. A medicinal composition according to any one of claims 39-42, which is produced by at least mixing, granulation, compression and heating.

44. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; said two or more sugar alcohols and/or saccharides comprise erythritol and trehalose; and a content of trehalose is from 0.3 to 50.0% by weight based on a content of erythritol.

45. A medicinal composition according to claim 44, wherein said heating is effected at 80 to 140°C.

46. A medicinal composition according to claim 44 or 45, wherein said heating is effected for 3 to 90 minutes.

47. A medicinal composition according to claim 44, wherein said active ingredient is contained such that its content falls within a range of from 0.05 to 25 wt.% based on a whole amount of said medicinal composition; and said medicinal composition has a wetting time of 10 seconds or shorter when tested by a wet test, disintegrates in 30 seconds when taken in the oral cavity, and has a hardness of 2 kp or higher.

48. A medicinal composition according to any one of claims 44-47, which is produced by at least mixing, granulation, compression and heating.

49. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; said two or more sugar alcohols and/or saccharides comprise erythritol and one of xylitol and sorbitol, and a content of one of xylitol and sorbitol is from 0.3 to 50.0% by weight based on a content of erythritol.

50. A medicinal composition according to claim 49, wherein said heating is effected at 80 to 140°C.

51. A medicinal composition according to claim 49 or 50, wherein said heating is effected for 3 to 90 minutes.

52. A medicinal composition according to claim 51, wherein said active ingredient is contained such that its content falls within a range of from 0.05 to 25 wt.% based on a whole amount of said medicinal composition; and said medicinal composition has a wetting time of 10 seconds or shorter when tested by a wetting test, disintegrates in 30 seconds when taken in the oral cavity, and has a hardness of 2 kp or higher.

53. A medicinal composition according to any one of claims 49-52, which is produced by at least mixing, granulation, compression and heating.

54. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; said two or more sugar alcohols and/or saccharides comprise mannitol and one of xylitol, sorbitol and trehalose; and a content of any one of xylitol, sorbitol and trehalose is from 0.3 to 50.0% by weight based on a content of mannitol.

55. A medicinal composition according to claim 54, wherein said heating is effected at 80 to 140°C.

56. A medicinal composition according to claim 54 or 55, wherein said heating is effected for 3 to 90 minutes.

57. A medicinal composition according to claim 56, wherein said active ingredient is contained such that its content falls within a range of from 0.05 to 25 wt.% based on a whole amount of said medicinal composition; and said medicinal composition has a wetting time of 10 seconds or shorter when tested by a wetting test, disintegrates in 30 seconds when taken in the oral cavity, and has a hardness of 2 kp or higher.

58. A medicinal composition according to any one of claims 54-57, which is produced by at least mixing, granulation, compression and heating.

59. A medicinal composition produced by at least mixing, compression and heating, wherein said medicinal composition comprises two or more of sugar alcohols and/or saccharides, and an active ingredient; a difference between a melting point of one having a highest content of said two or more sugar alcohols and/or saccharides and that of any remaining one of said two or more sugar alcohols and/or saccharides is 5°C or greater; said two or more sugar alcohols and/or saccharides comprise lactose and sorbitol; and a content of sorbitol is from 0.3 to 50.0% by weight based on a content of lactose.

60. A medicinal composition according to claim 59, wherein said heating is effected at 80 to 140°C.

61. A medicinal composition according to claim 59 or 60, wherein said heating is effected for 3 to 90 minutes.

62. A medicinal composition according to claim 61, wherein said active ingredient is contained such that its content falls within a range of from 0.05 to 25 wt.% based on a whole amount of said medicinal composition; and said medicinal composition has a wetting time of 10 seconds or shorter when tested by a wetting test, disintegrates in 30 seconds when taken in the oral cavity, and has a hardness of 2 kp or higher.

63. A medicinal composition according to any one of claims 1-62, which is in a form of a tablet.

64. A medicinal composition according to any one of claims 1-62, which is in a form of a tablet which shows a hardness of 0.5 kp or higher when its diameter or maximum length is less than 8 mm, a hardness of 1 kp or higher when its diameter or maximum length is 8 mm or more but less than 10 mm, a hardness of 2 kp or higher when its diameter or maximum length is 10 mm or more but less than 15 mm, a hardness of 3 kp or higher when its diameter or maximum lengthy is 15 mm or more but less than 20 mm, or a hardness of 4 kp or higher when its diameter or maximum length is 20 mm or more.

65. A process for the production of a medicinal composition, comprising steps of preparing a mixture of an active ingredient and two or more of sugar alcohols and/or saccharides, compressing said mixture into a compact and heating said compact, wherein a difference between a melting point of one having a highest content of said two or more sugar alcohols and/or saccharides and that of any remaining one of said two or more sugar alcohols and/or saccharides is 5°C or greater.

66. A process according to claim 65, wherein said compression is tableting.

67. A process according to claim 66, wherein said tableting is effected at a compressing pressure of from 300 to 1,500 kgf.

68. A process according to any one of claims 65-67, wherein said heating is effected at 80 to 140°C.

69. A process according to any one of claims 65-68, wherein said heating is effected for 3 to 90 minutes.

70. A process according to any one of claims 65-69, wherein said heating is effected in an air-convection constant-temperature oven, constant-temperature drying oven, vacuum drying oven or microwave oven.

71. A process according to any one of claims 65-70, wherein said mixture of said two or more sugar alcohols and/or saccharides is granulated before compression.
